# EUROPEAN PATENT SPECIFICATION

(11) **EP 0 638 174 B1**
(45) Date of publication and mention of the grant of the patent: **23.07.1997**
(21) Application number: 93910753.8
(22) Date of filing: 23.04.1993
(51) Int. Cl.: G01N 33/569, C07K 14/12, C12N 15/86

(54) **MEASLES VIRUS-SPECIFIC ANTIBODY DETECTION USING RECOMBINANT MEASLES PROTEINS**
NACHWEIS VON MASERVIRUS-SPECIFISCHER ANTIKÖRPERN DURCH GEBRAUCH VON REKOMBINANTEN MASERPROTEINEN
DETECTION D'ANTICORPS SPECIFIQUES AU VIRUS MORBILLEUX AU MOYEN DE PROTEINES MORBILLEUSES RECOMBINEES

(30) Priority: 24.04.1992 US 873017
(43) Date of publication of application: 15.02.1995
(73) Proprietor: THE GOVERNMENT OF THE UNITED STATES OF AMERICA as represented by the SECRETARY OF THE DEPARTMENT OF HEALTH AND HUMAN SERVICES, Bethesda, MD 20892-9902 (US)
(72) Inventor: BELLINI, William, J., Lilburn, GA 30247 (US); HUMMEL, Kimberly, B., Avondale Estates, GA 30002 (US); HEATH, Janet, L., Decatur, GA 30030 (US); ERDMAN, Dean, Atlanta, GA 30333 (US)
(74) Representative: Perry, Robert Edward
(86) International application number: US9303833
(87) International publication number: WO9322683

(56) References cited:
- EP-A- 0 265 785
- EP-A- 0 279 661
- JOURNAL OF CLINICAL MICROBIOLOGY vol. 30, no. 11, November 1992, pages 2874 - 2880 K.B.HUMMEL ET AL. 'Baculovirus Expression of the Nucleoprotein Gene of Measles Virus and Utility of the Recombinant Protein in Diagnostic Enzyme Immunoassays'
- JOURNAL OF VIROLOGY vol. 65, no. 4, April 1991, pages 1695 - 1700 B.BANKAMP ET AL. 'Measles Virus Nucleocapsid Protein Protects Rats from Encephalitis'
- JOURNAL OF VIROLOGY vol. 64, no. 1, January 1990, pages 37 - 50 J.VIALARD ET AL. 'Synthesis of the Membrane Fusion and Hemagglutinin Proteins of Measles Virus, Using a Novel Baculovirus Vector Containing the Beta-Galactoside Gene' cited in the application
- JOURNAL OF CLINICAL MICROBIOLOGY vol. 29, no. 7, July 1991, pages 1466 - 1471 D.D.ERDMAN ET AL. 'Evaluation of Monoclonal Antibody-Based Capture Enzyme Immunoassays for Detection of Specific Antibodies to Measles Virus.' cited in the application
- THE JOURNAL OF GENERAL VIROLOGY vol. 68, no. 5, May 1987, pages 1233 - 1250 Y. MATSUURA ET AL. 'Baculovirus Expression Vectors: the Requirements for High Level Expression of Proteins, Including Glycoproteins' cited in the application

## Description

### FIELD OF THE INVENTION

The present invention relates to the detection of measles-specific IgG and IgM antibodies using measles antigens produced by recombinant DNA techniques. The expressed measles antigens maintain reactivity with immunoglobulins from patients infected with measles and are produced by expressing cloned genes in alternative hosts. These antigens are then used in place of whole measles virus preparations or derivatives thereof in assays to detect anti-measles antibodies in patients potentially exposed to the virus. The antigens preferably are immobilized on a surface, directly or indirectly as in sandwich assays, and then incubated with a serum sample. Antigen-binding immunoglobulins in the serum are detected by labelled antibody binding reagents such as anti-human immunoglobulin secondary antibodies. A recombinant baculovirus system is preferred for production of measles antigens, and the nucleoprotein gene of measle virus expressed in such a system is particularly preferred in the assays of the invention.

### BACKGROUND OF THE INVENTION

Measles is a major health problem in the world. This is true especially in developing countries, which worldwide report an estimated 70 million cases each year that cause the death of 2 million children.

Within the United States, eradication of measles remains elusive despite the implementation of vaccine regimens since 1963. As a result of vaccine failure or failure to vaccinate, over 27,000 measles cases were reported in 1990 in the U.S., 50% more than in 1989 and nearly ten times the number reported in 1988. Centers for Disease Control (1990) *Morbid. Mortal. Weekly Rep.* 39: 353-363.

Due to the continuing worldwide problem of measles and the resurgence of measles in the United States (together with its frequent complications), there is a continuing need for improved methods of preventing and treating measles, and concomitantly for improved methods of diagnosis.

Current technology for measles virus detection includes the use of whole virus as antigen in an enzyme immunoassay format. Commercial diagnostic tests such as "MEASELISA" and "MEASLESTAT M," supplied by Whittaker Bioproducts, Inc., are available for the detection of IgG and IgM antibodies, respectively, and employ the Edmonston vaccine strain of measles virus grown in MK-2 cells as whole virus antigen. Boteler *et al.* (1983) *J*. *Clin. Microbiol.* 17: 814-818.

A capture IgM EIA of similar design has recently been described that also uses the Edmonston strain, grown in E-6 Vero cells, as an assay antigen. Erdman *et al.* (1991) *J. Clin. Microbiol.* 29*:* 1466-1471. The whole virus capture EIA differs from the "MEASLESTAT M" test in that patient serum in the "MEASLESTAT M" test must first be pretreated to remove interfering IgG antibodies, whereas in the capture EIA, serum IgM simply is "captured" using an anti-human IgM antibody. Thus, in the absence of serum pretreatment, the capture EIA may be somewhat easier to use.

Although available enzyme immunoassays (EIAs) are relatively sensitive and practical, all of them rely on whole virus from cell culture. Unfortunately, measles virus grown in culture generally yields a low titer, highly variable reagent. The difficulty in routinely obtaining large quantities of pure measles virus adds greatly to the cost and reduces the practicality of measles diagnostic assays. In addition to the inherent variability in using whole virus antigen, proper facilities and safeguards must be employed in the handling of infectious material. Thus, there is a need for safer and more economical and convenient methods of producing large quantities of virus or viral antigens suitable for diagnostic applications.

One alternative to using whole virus as antigen is the production of recombinant proteins in heterologous host cells via the expression of cloned genes. Production by such systems would overcome the necessity for whole measles virus in diagnostic EIAs by providing a virtually unlimited source of measles antigens suitable for use in measles EIAs. It would also provide sufficient antigen for evaluating rates of seroconversion and seroprevelance in vaccinated populations. Moreover, expressing proteins by recombinant means would also allow for lower batch to batch variation and greater ease in calibrating and quantitating assays.

Unfortunately, the potential advantages of recombinant antigen production have not been realized heretofore in the context of measles detection. Proteins expressed in prokaryotic and simple eukaryotic systems, such as yeast, often display immunological characteristics different from their natural counterparts. Differences like these can limit the utility of recombinant proteins in assays that require recognition of serum antibodies that bind to native protein. To circumvent such problems, recombinant baculoviruses were employed for the high-level expression of genes in higher eukaryotes. Success in constructing vectors that produce biologically active protein products has been achieved for other proteins, and studies of these proteins show that post-translational modifications are similar in insect and mammalian cells, although glycosylation varies in detail.

Although the fusion and hemagglutinin proteins of measles virus have been expressed by such recombinant means in a baculovirus system, proper glycosylation is impaired. Vialard et al. (1990) *Journal of Virology* 64: 37-50.

Bancamp *et al,* J. Virol. 65(4):1695-1700 (1991) disclose an enzyme-linked immunosorbent assay for the determination of measles antibodies in serum samples of MV-infected animals. In this method, whole purified MV virions are used as coat antigen. The animals are immunised with recombinant vaccinia virus expressing the nucleocapsid (N) protein of measles virus.

### SUMMARY OF THE INVENTION

According to a first aspect of the present invention, an immunoassay method, for the detection of measles-specific immunoglobulin in a human serum sample, comprises the steps of:
(i) contacting the sample with an immobilised recombinant measles nucleoprotein antigen, under conditions that permit binding of the immunoglobulin an the immobilised antigen; and
(ii) detecting the presence of measles-specific immunoglobulin bound to the immobilised antigen.

According to a second aspect of the invention, a capture immunoassay method, for the detection of measles-specific immunoglobulin IgM in a human serum sample, comprises the steps of:
(i) contacting the sample with immobilised purified anti-human IgM capture antibody, under conditions that permit binding of the IgM and the immobilised antibody;
(ii) contacting the captured IgM with a recombinant measles nucleoprotein, under conditions sufficient to permit binding of the nucleoprotein and the captured IgM; and
(iii) detecting the presence of nucleoprotein bound to the captured IgM.

By comparison with the procedure disclosed by Vialard *et al,* supra, the nucleoprotein, which is not glycosylated, when produced by expression in baculovirus in accordance with the present invention, more closely resembles the authentic viral protein.

In preferred embodiments of the invention, the measles antigen is expressed via a recombinant DNA encoding the antigen, wherein the recombinant DNA comprises a baculovirus polyhedrin promoter operably linked to a cDNA encoding the antigen and expression of the antigen by the promoter is in *Spodoptera frugiperda* cells. Particularly preferred in this aspect of the invention, is the plasmid pAcYM1S-MVN.

Other objects, features and advantages of the present invention will become apparent from the following detailed description. It should be understood, however, that the detailed description and the specific examples, while indicating preferred embodiments of the invention, are given by way of illustration only.

### BRIEF DESCRIPTION OF THE DRAWINGS

FIGURE 1 shows a comparison of indirect IgG EIA results using a recombinant N protein or whole measles virus as antigen to assay 160 serum specimens from persons with and without prior exposure to measles virus or vaccine. Line fitted by linear regression (R: = 0.80).

FIGURE 2 shows a comparison of capture IgM EIA results using a recombinant N protein or whole measles virus as antigen to assay 25 acute-phase (○) and convalescent-phase (●) serum pairs from persons with clinical measles infection. Line fitted by linear regression (R² = 0.85).

FIGURE 3 is a schematic diagram of the subcloning strategy for the insertion of a full-length nucleoprotein gene of measles virus into the baculovirus expression vector pAcYMlS. Restriction endonuclease digests were performed as indicated. Hatched and stippled areas represent coding regions of the measles nucleoprotein and the baculovirus polyhedrin gene, respectively.

FIGURE 4 is a composite autoradiogram showing the results of immunoprecipitation experiments that demonstrate that the recombinant nucleoprotein has the same electrophoretic mobility and antigenicity as the authentic nucleoprotein.

FIGURE 5 is a schematic diagram showing the ligation junctions of measles fusion and hemagglutinin genes cloned into the baculovirus expression vector pAcYM1S.

### DETAILED DESCRIPTION OF THE INVENTION

The present invention overcomes many of the disadvantages of the conventional methodology for detecting measles-specific antibodies by allowing one to readily make recombinant measles antigens that serve as more sensitive detectors in enzyme immunosorbent ["EIAs"] and other immunological assays.

In general, the method of the invention involves constructing a plasmid DNA, or the like, in which a DNA sequence that encodes a protein of antigenic potential is under the control of appropriate *cis*-acting control signals for transcription so that, when the plasmid DNA is introduced into appropriate host cells, and the proper conditions for expression are provided, the antigen is produced in the transformed cells.

Methods for cloning genes, for manipulating the genes to form expression vectors, and for expressing the protein encoded by the gene in a heterologous host are well-known, and it will be appreciated that these techniques can be used in accordance with the invention to provide the expression vehicles, host cells, and the like, for expressing cloned genes encoding measles antigens in a host to produce recombinant antigens for use in diagnostic assays, *inter alia.* See, for instance, Sambrook *et al.* MOLCULAR CLONING, A LABORATORY MANUAL, Second Edition, Vol. 1-3 (Cold Spring Harbor Laboratory, 1989), and references cited below.

The measles virus genome is composed of about 16,000 nucleotides of RNA of the negative polarity, that is, the genome does not serve as messenger RNA but must be transcribed into complementary monocistronic messages for viral protein synthesis. The gene order of the measles genome is 3' N, P/V/C, M, F, HA, L 5'. The N gene encodes the nucleoprotein, or N protein. The P, V, C gene encodes three proteins, the phosphoprotein, V protein, and C protein, respectively, within the same nucleotide sequence, by RNA editing or accession of overlapping open reading frames. The M gene encodes the matrix (or membrane) protein, which is involved in virus assembly and budding. The F and HA genes encode the fusion and hemagglutinin proteins, respectively, which are transmembrane glycoproteins involved in envelope fusion and host-cell recognition. The L gene encodes the large polymerase protein. There appears to be only one promoter in the measles virus genome, located at the 3' end of the genome, and the synthesis of messenger RNAs (mRNAs) forms a gradient wherein N mRNAs are the most abundant and L mRNAs are the least abundant. Thus, in infected cells the N protein is the most abundant protein, and the immune response to this protein occurs early in infection. For these reasons, a recombinant source of the N protein was deemed desirable for purposes of developing an ELISA assay for detection of antibody to measles virus. Antigens suitable for use in the invention include any measles protein that combines with any measles-specific antibody of a patient exposed to or infected with measles virus. Preferred antigens of the invention include those that predominantly engender the immune response in patients exposed to or infected with the measles virus, which, therefore, typically are recognized most readily by antibodies of a patient. Particularly preferred antibodies of the invention include the membrane fusion and hemagglutinin proteins of measles virus. Among the most highly preferred antigens is the measles nucleoprotein which has been identified as a major target of the human T-cell response.

It will be furthermore appreciated that the antigens can be the native antigens or can be modified versions thereof. Well known techniques of molecular biology can be used to alter the amino acid sequence of a measles antigen to produce modified versions of the antigen that may be used in accordance with the invention.

These techniques are useful to alter patterns of post-translational modification. For instance, changes in the amino acid sequence of a protein can alter its glycosylation or phosphorylation pattern. Such techniques are also useful to provide specific functional moieties that aid efficient expression or purification of recombinantly expressed proteins, *inter alia.* It will be understood, therefore, that, in accordance with the invention, the entire panoply of recombinant DNA techniques can be employed to provide antigens useful to detecting anti-measles immunoglobulins.

A variety of expression systems may be used to produce measles antigens in accordance with the invention. For instance, a variety of expression vectors suitable to producing proteins in *E*. *coli, B. subtilis,* yeast, insect and mammalian cells have been described, any of which might be used in accordance with the invention to produce a measles antigen suitable to detecting anti-measles antibodies in exposed patients. However, whereas expression in more rudimentary systems such as bacteria and yeast yield high levels of protein, post-translational modifications important to antigenicity often are not performed. In contrast, mammalian systems which maintain the integrity of the protein, often result in low yields. Among the expression systems of the invention the baculovirus expression system, which can be used to express large quantities of a foreign protein and can provide necessary processing, is therefore highly preferred. Particularly preferred in this regard is the baculovirus expression system that utilizes the polyhedrin promoter to direct expression of measles antigens. Matsuura *et al.* (1987) *J. Gen. Virol.* 68: 1233-1250.

Antigens produced in accordance with the invention can be used in a variety of immunological assays to detect anti-measles antibodies in a patient. In fact, it will be readily appreciated by those of ordinary skill that antigens according to the invention can be used in place of natural virus in practically any immunological assay for detection of measles-specific antibodies.

The assays include direct and indirect assays, sandwich assays, solid phase assays such as those using plates or beads among others, and liquid phase assays, *inter alia.* Assays suitable for use in the invention include those that use primary and secondary antibodies, and those that use antibody binding reagents such as protein A. Moreover, a variety of detection methods can be used in the invention, including colorimetric, fluorescent, phosphorescent, chemiluminescent, luminescent and radioactive methods.

The present invention is further described by reference to the following, illustrative examples.

### EXAMPLE 1: Recombinant measles nucleoprotein production

Two cDNA clones, each encoding part of the nucleoprotein gene of measles virus (Edmonston vaccine strain), were ligated together to generate a full-length open reading frame suitable for insertion into the baculovirus expression vector pAcYM1S.

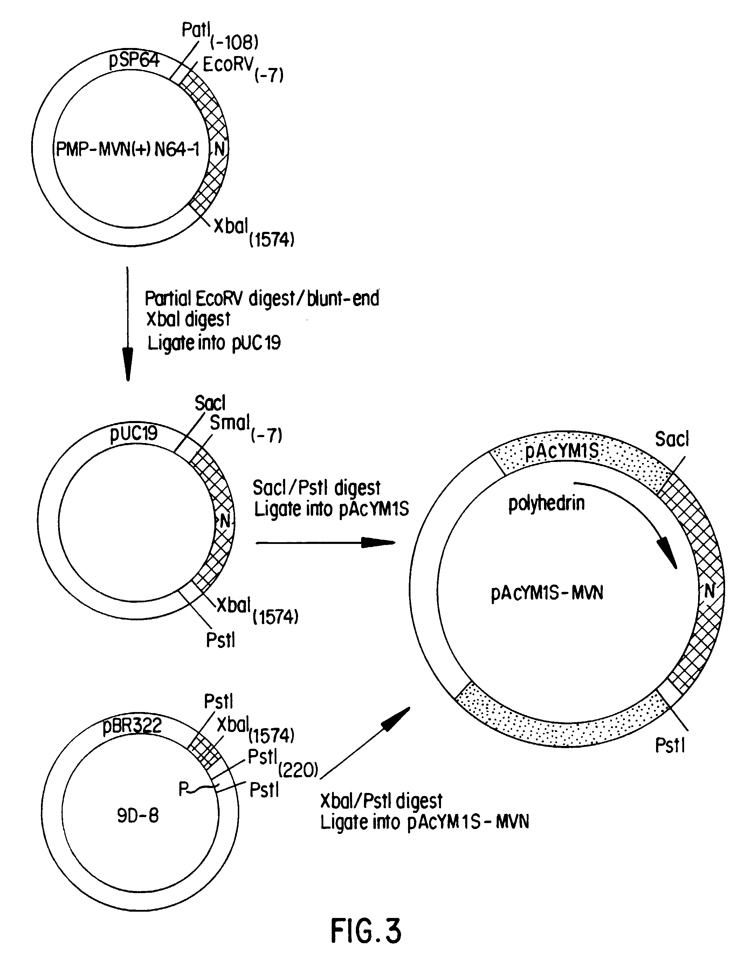

The full-length nucleoprotein cDNA insert was 1.855 kb in length, originating 7 basepairs upstream from the first in-frame AUG, and extending through the stop codon, intergenic region, and the first 200 nucleotides of the P gene, as shown in FIGURE 3.

Transfection of *Autographa californica* nuclear polyhedrosis virus (AcNPV) and vector DNA into cultured *Spodoptera frugiperda* cells (Sf9) was achieved using the calcium phosphate precipitation technique, as described by Summers and Smith (1987) Texas Agricultural Experiment Station. Bulletin No. 1555

One round of limiting dilutions, followed by selection based on dot blot hybridizations using a nick-translated cDNA probe of the nucleoprotein gene, was used to isolate a recombinant baculovirus which was purified by two rounds of plaquing. Each round of successive screening was confirmed by positive immunofluorescence as observed in acetone-fixed cells using mouse monoclonal antibodies to the nucleoprotein and a fluorescein isothiocyanate conjugated secondary antibody.

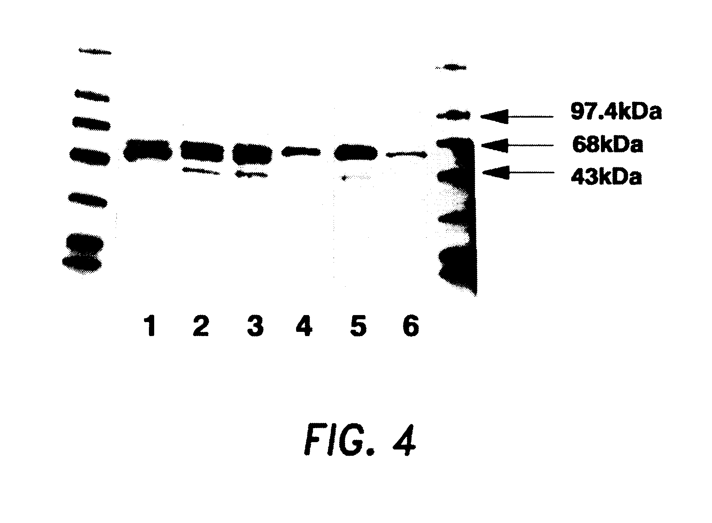

Radioimmune precipitation experiments were carried out to compare the properties of the nucleoprotein obtained by expression in a recombinant baculovirus with the authentic viral protein. Radiolabeled, infected cell lysates were immune precipitated with nucleoprotein-specific monoclonal antibodies and immune complexes were resolved by SDS-PAGE through a 4-20% gradient gel and visualized by fluorography. The results of these experiments are shown in FIGURE 4.

Lane 1 shows the immunoprecipitated material obtained when lysates of Sf9 cells infected with the recombinant baculovirus expressing the nucleoprotein were radiolabeled with [³²P]orthophosphoric acid and immune precipitated with two nucleoprotein specific monoclonal antibodies, 81I168 and 83VIIKK2.

Lanes 2 and 3 show the immunoprecipitated material obtained when lysates of Sf9 cells infected with the recombinant baculovirus expressing the nucleoprotein were radiolabeled with [³⁵S]methionine and immune precipitated with nucleoprotein-specific monoclonal antibodies 81I168 or 83VIIKK2, respectively.

Lanes 4 and 5 show the immunoprecipitated material obtained when Edmonston measles virus-infected CV-1 cell lysates were radiolabeled with [³⁵S]methionine and immune precipitated with nucleoprotein-specific monoclonal antibodies 81I168 and 83VIIKK2, at 5 and 20 hour exposure times, respectively.

Lane 6 shows the immunoprecipitated material obtained when an Edmonston measles virus-infected CV-1 cell lysate radiolabeled with [³⁵S]methionine was immune precipitated with an antiserum generated in mouse against the baculovirus produced measles nucleoprotein.

The recombinant nucleoprotein comigrated with that of the Edmonston vaccine strain on SDS-PAGE, and it was phosphorylated. It was reactive with a panel of monoclonal antibodies in both immuno-fluorescent and radio immune precipitation assays and it was immunogenic, eliciting antisera in mice that recognized the native nucleoprotein. In sum, the nucleoprotein obtained from the Edmonston strain of measles virus was indistinguishable from that obtained by expressing a cloned nucleoprotein gene in the baculovirus expression system (with regard to electrophoretic mobility and the maintenance of epitopes as defined by antibody recognition).

### EXAMPLE 2: Indirect anti-measles IgG EIA using a recombinant nucleoprotein.

An indirect IgG EIA using the recombinant nucleoprotein as antigen was performed by modification of assays using the Edmonston strain of measles virus as previously described by Erdman *et al*. (1990), *J. Clin., Microbiol.* 29: 1466-1471.

For antigen preparation, Sf9 cells were infected at a MOI = 1 with the recombinant baculovirus and harvested 72 hours postinfection. The cell suspension was washed twice in PBS, pH 7.2, adjusted to a cell density of 5.0 x 10⁶ cells/ml, and freeze-thawed three times. Large cellular debris was pelleted by low speed centrifugation (500 x g for 15 min.) and the supernatant was collected and stored at - 70°C until use. Uninfected cells were processed similarly for negative control antigen.

75 µls of a freeze-thaw lysate was used to coat microtiter plates, such as "IMMULON II" plates, at dilutions ranging from 1:100 to 1:1000. An uninfected cell lysate was run in duplicate wells and served as a negative control. After an incubation of 1.5 hours,. plates were washed five times with 0.01 M PBS, pH 7.2 / 0.05% Tween 20, ["PBS/T"]. Test sera was diluted 1:100 in 0.01 M PBS, pH 7.2 / 0.5% Gelatin / 0.15% Tween 20 ["PBS/GT"] supplemented to 4% with normal goat sera, such as that obtained from "DIFCO" and to 10% with uninfected Sf9 cells, and was incubated in triplicate wells for 1 hour at 37°C. A standard serum titration was included in each run.

Plates were washed three times with PBS/T. Goat anti-human IgG horseradish peroxidase was diluted 1:3000 to 1:5000 in PBS/GT, added to wells, and incubated for 1 hour at 37°C. The plates were then washed three times with PBS/T, incubated for 15 minutes at room temperature with the enzyme substrate TMB, 3,3',5,5' tetramethylbenzidine, such as that obtained from "SIGMA", and the reaction stopped with 75 µls of 2 M phosphoric acid. Colorimetric readings were measured at 450nm using an automated microtiter plate reader, in this case a Dynatech MR5000 reader.

Results for the assay were expressed as P-N values, defined as the average difference in measured absorbance values between duplicate wells of nucleoprotein ("P") and normal Sf9 cells ("N") for each serum specimen.

The cutoff value for a positive test was taken as the mean plus 3 standard deviations of a panel of 20 control specimens with no detectable plaque neutralizing antibodies. The cutoff chosen for the indirect IgG assay using the recombinant nucleoprotein was P-N = 0.09. P/N ratios ≥ 3 were also required, to account for occasional specimens with high background signal.

The recombinant nucleoprotein in the indirect IgG EIA detected measles-specific antibodies in 99% of serum specimens (129/131) which were positive by whole virus EIA. These results were better correlated with the presence of neutralizing antibodies than the results obtained using a commercial EIA to assay the same specimens, particularly at lower antibody titers. Moreover, 27 specimens without detectable IgG antibodies by whole virus EIA were also negative by the N recombinant EIA.

### EXAMPLE 3: Capture anti-measles IgM EIA using a recombinant nucleoprotein.

A capture IgM EIA using the recombinant nucleoprotein as antigen was performed by modification of assays using the Edmonston strain of measles virus, as previously described by Erdman *et al.* (1990) *J. Clin., Microbiol.* 29: 1466-1471.

Affinity purified anti-human IgM capture antibody, such as that obtained from "ORGANON TEKNIKA CORP.", was diluted 1:800 in 0.01 M.PBS, pH 7.2 and 75 µls were added to wells of a microtiter plate, such as "IMMULON II" plates. After a 1 hour incubation at 37°C in a humidity chamber the plates were washed three times with PBS/T. 75 µls of test serum diluted to 1:200 in PBS/GT was added to triplicate wells and incubated for 1 hour at 37°C. The plates were then washed three times in PBS/T.

The freeze-thaw lysate diluted to 1:250 in PBS/GT supplemented with 4% normal goat serum and 0.3% sodium deoxycholate, was added to the wells and incubated for 2 hours at 37°C. Uninfected cell lysate served as a negative control and was run in duplicate wells.

The plates were then washed three times in PBS/T and incubated for 1 hour at 37°C with 75 µls of a biotinylated anti-nucleoprotein monoclonal antibody diluted 1:5000 in PBS/GT. 75 µls of streptavidin peroxidase, such as that obtained from "AMERSHAM INTERNATIONAL," diluted 1:3000 in PBS/GT was added and incubated for 20 minutes at 37°C.

The plates were then washed five times in PBS/T, incubated for 15 minutes at room temperature with the enzyme substrate TMB, 3,3',5,5' tetramethylbenzidine, as, for instance obtained from "SIGMA", and the reaction stopped with 75 µls of 2 M phosphoric acid. Colormetric readings were measured at 450nm using a Dynatech MR5000 reader.

Results for the assay were expressed as P-N values, defined as the average difference in measured absorbance values between duplicate wells of nucleoprotein ("P") and normal Sf9 cells ("N") for each serum specimen. The cutoff value for a positive test was taken as the mean plus 3 standard deviations of a panel of 20 control specimens with no detectable plaque neutralizing antibodies. The cutoff chosen for the capture IgM assay using the recombinant Nucleo protein was P-N = 0.21. P/N ratios ≥ 3 were also required to account for occasional specimens with high background signal.

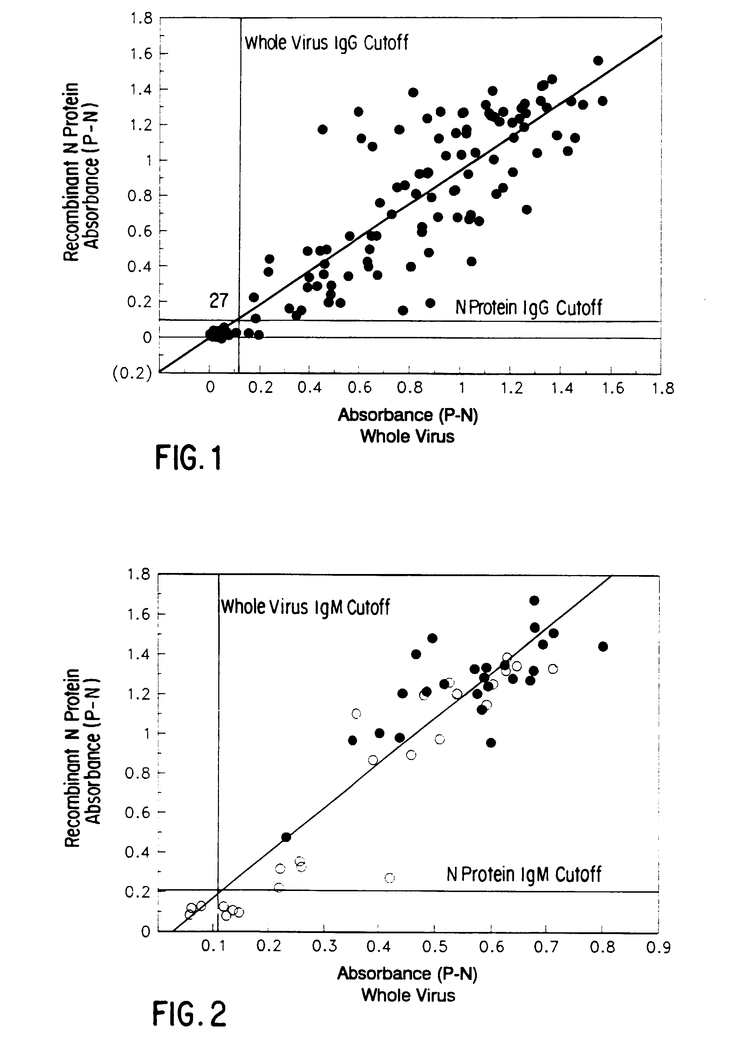

The sensitivities of the capture IgM EIAs using the recombinant nucleoprotein and whole measles virus were compared using 25 acute- and convalescent-phase serum pairs from persons with clinical measles virus infection (FIGURE 2). IgM antibodies were detected by the recombinant nucleoprotein EIA in 18 (72%) acute-phase specimens. Twenty-two (88%) acute-phase specimens, including the 18 specimens positive by the recombinant nucleoprotein EIA, were positive with whole virus. All convalescent-phase specimens were positive for IgM antibodies by both assays. The specificity of the recombinant nucleoprotein capture EIA was determined by testing 120 serum specimens from healthy persons and persons with other paromyxovirus infections. Of these, one person (<1%) had detectable IgM antibodies by both the recombinant nucleoprotein and whole virus EIAs.

### EXAMPLE 4: An anti-measles IgG EIA was more accurate using a recombinant nucleoprotein than whole measles virus as antigen.

One hundred and sixty serum specimens collected from persons with and without a history of prior exposure to measles virus or vaccine were tested simultaneously for IgG antibodies by the N recombinant and whole virus EIAs as shown in FIGURE 1. Specific IgG antibodies were detected by the N recombinant EIA in 129 of 131 (99%) specimens positive by the whole virus EIA. All 27 specimens without detectable IgG antibodies by whole virus EIA were also negative by recombinant EIA.

Two hundred and sixty-eight serum specimens previously tested for plaque neutralizing antibodies by Paul Albrecht, Food and Drug Administration, were also tested for specific IgG antibodies by the N recombinant EIA and by commercial EIA ("MEASELISA"). See Albrecht *et al.* (1981) *J. Virol. Methods* 3*:* 251-260 and Orenstein *et al.* (1987) *J. Infect. Dis.* 155*:* 146-149.

As shown in TABLE 1, N recombinant EIA values were generally in better agreement with the presence of neutralizing antibodies than the values obtained by commercial EIA, particularly at lower levels of neutralizing antibodies.

Indeed, 48 of 109 (44%) specimens with neutralizing antibody titers ≤ 120 were positive by N recombinant EIA as compared with only 11 (10%) positive by commercial EIA. Overall, 137 of 223 (61%) specimens positive for neutralizing antibodies were positive by N recombinant EIA, and 88 (40%) were positive by commercial EIA. Of the 45 specimens without detectable neutralizing antibodies, 1 (2%) was positive by each assay.

**TABLE 1.**

| Comparison of recombinant N protein and commercial IgG EIAs for determination of measles antibody status of 268 persons previously testeu for measles neutralizing antobodies. | | | | | | | |
|---|---|---|---|---|---|---|---|
| Neutralizing Antibody Titers² | No. Tested | EIQ Results¹ B-MVN Protein/Commercial | | | | Recombinant N Protein Total + | Commercial Total + |
| | | +/+ | +/- | -/+ | -/- | | |
| <8 | 45 | 0 (0%) | 1 (2%) | 1 (2%) | 43 (96%) | 1 (2%) | 1 (2%) |
| 8-60 | 50 | 4 (8%) | 14 (28%) | 1 (2%) | 31(62%) | 18 (36%) | 5 (10%) |
| 61-120 | 59 | 5 (8%) | 25 (42%) | 1 (2%) | 28 (47%) | 30 (50%) | 6 (7%) |
| 121-240 | 42 | 10 (24%) | 13 (31%) | 0 (0%) | 19 (45%) | 23 (55%) | 10 (24%) |
| >240 | 72 | 63 (88%) | 3 (4%) | 4 (6%) | 2 (3%) | 66 (92%) | 67 (94%) |

| | | | | | | | |
|---|---|---|---|---|---|---|---|
| ¹ Specimens tested by recombinant N protein and commercial (MEASELISA II Test Kit, Whittaker Bioproducts, Inc.) IgG EIAs. Results expressed as positive (+) or negative (-) for measles-specific IgG antibodies. | | | | | | | |
| ² Plaque neutralization assay performed by Paul Albrecht, Food and Drug Administration. | | | | | | | |

### EXAMPLE 5: Potential for EIAs that use recombinant measles fusion and hemagglutinin proteins produced by baculovirus expression.

The hemagglutinin and fusion proteins are the two transmembraneous envelope glycoproteins of the measles virion. The hemagglutinin is responsible for host cell attachment and the agglutination of erythrocytes. The fusion protein is necessary for viral entry and hemolysis. Neutralizing antibodies against both proteins play a role in establishing life-long immunity. See Vol. 1, pages 1013-1044 in VIROLOGY, 2nd Ed., Fields *et al*., Eds., Raven Press, New York (1990).

The expression of these glycoproteins in a baculovirus system would also provide a source of recombinant antigens for use in assays for detection of measles-specific antibodies. Moreover, their use in combination with the nucleoprotein, for instance, could enhance the sensitivity of enzyme immunoassays in the detection of antibodies against measles virus.

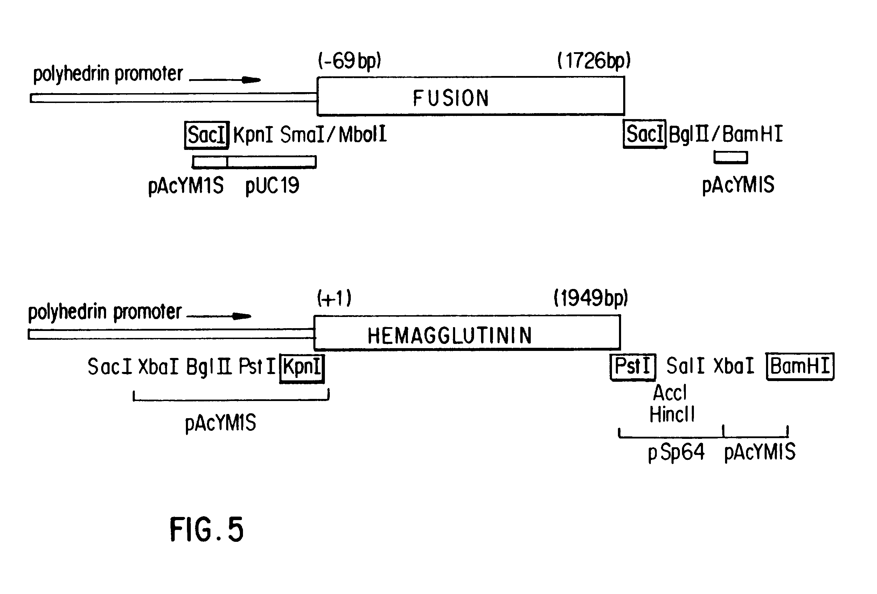

To obtain the glycoproteins by baculovirus expression the genes for the hemagglutinin and fusion proteins were first cloned into a suitable expression vector. A 1.949 kb cDNA clone representative of the hemagglutinin gene of the Edmonston vaccine strain was ligated into the baculovirus transfer vector pAcYM1S to provide a full-length open reading frame downstream of the transcriptional leader sequences of the polyhedrin gene (FIGURE 5).

Similarly, two partial cDNA clones encoding the fusion gene were ligated together to produce a full-length coding region, 1.726 kb in length, which was suitable for expression (FIGURE 5).

Transfection of AcNPV and vector DNA into cultured Sf9 cells was achieved by the calcium phosphate precipitation technique following procedures described by Summers and Smith (1987) Texas Agricultural Experiment Station. Bulletin No. 1555A.

After two rounds of limiting dilution cloning and dot blot hybridizations to measles virus gene sequences, recombinant baculoviruses were isolated and plaque-purified. Each round of successive screening was confirmed by positive immunofluorescence observed in acetone-fixed cells using mouse monoclonal antibodies and a fluorescein isothiocyanate conjugated secondary antibody. Following radioimmune precipitation, the recombinant glycoproteins migrated similarly with that of Edmonston on SDS-PAGE, although with apparent differences in glycosylation, as previously characterized by Vialard *et al.* (1990) *Journal of Virology* 64: 37-50.

Relatively low levels of expression of the glycoproteins as compared to the nucleoprotein was observed, due to extensive post-translational modifications. In consequence of the low level of expression in preliminary EIAs using cell lysates the recombinant glycoproteins did not provide signal to background ratios high enough for highly accurate assays.

For use in EIAs, therefore, it may be desirable to further purify and concentrate these recombinant proteins prior to use. A variety of purification techniques that may be used for this purpose are well known, including but not limited to various kinds of affinity chromatography.

## Claims

1. An immunoassay method, for the detection of measles-specific immunoglobulin in a human serum sample, comprising the steps of:
(i) contacting the sample with an immobilised recombinant measles nucleoprotein antigen under conditions that permit binding of the immunoglobulin and the immobilised antigen; and
(ii) detecting the presence of measles-specific immunoglobulin bound to the immobilised antigen.

2. A capture immunoassay method, for the detection of measles-specific immunoglobulin IgM in a human serum sample, comprising the steps of:
(i) contacting the sample with immobilised purified anti-human IgM capture antibody, under conditions that permit binding of the IgM and the immobilised antibody;
(ii) contacting the captured IgM with a recombinant measles nucleoprotein, under conditions sufficient to permit binding of the nucleoprotein and the captured IgM; and
(iii) detecting the presence of nucleoprotein bound to the captured IgM.

3. The method of claim 2, wherein step (iii) comprises contacting the IgM-bound nucleoprotein with an anti-nucleoprotein monoclonal antibody bound to a detectable label, under conditions sufficient to permit binding therebetween, and detecting the presence of bound label.

4. The method of claim 2 or claim 3, wherein the capture antibody is immobilised by indirect fixation to a plate surface.

5. The method of claim 1, wherein the antigen is immobilised by indirect fixation to a surface.

6. The method of any preceding claim, wherein the nucleoprotein is expressed by a cell transfected with a recombinant DNA sequence comprising a baculovirus polyhedrin promoter operably linked to a DNA molecule encoding the nucleoprotein.

7. The method of claim 6, wherein the cell is Spodoptera frugiperda.

8. The method of claim 6, wherein the cell is transfected with a recombinant DNA sequence comprising plasmid pAcYM1S-MVN.

## Revendications

1. Procédé de dosage immunologique destiné à la détection d'immunoglobuline spécifique de la rougeole dans un échantillon sérique humain, comprenant les étapes consistant à:
(i) mettre l'échantillon en contact avec un antigène nucléoprotéique de la rougeole recombiné, immobilisé, dans des conditions permettant la liaison de l'immunoglobuline et de l'antigène immobilisé et
(ii) détecter la présence de l'immunoglobuline spécifique de la rougeole liée à l'antigène immobilisé.

2. Procédé de dosage immunologique par piégeage destiné à la détection d'immunoglobuline IgM spécifique de la rougeole dans un échantillon sérique humain, comprenant les étapes consistant à :
(i) mettre l'échantillon en contact avec un anticorps de piégeage anti-IgM humaine, purifié, immobilisé, dans des conditions permettant la liaison de l'IgM et de l'anticorps immobilisé ;
(ii) mettre l'IgM piégée en contact avec une nucléoprotéine recombinée de la rougeole dans des conditions suffisantes pour permettre la liaison de la nucléoprotéine et de l'IgM piégée et
(iii) détecter la présence de la nucléoprotéine liée à l'IgM piégée.

3. Procédé selon la revendication 2, dans lequel l'étape (iii) comprend la mise en contact de la nucléoprotéine liée à l'IgM avec un anticorps monoclonal anti-nucléoprotéine lié à un marqueur détectable, dans des conditions suffisantes pour permettre leur liaison, et la détection de la présence du marqueur lié.

4. Procédé selon la revendication 2 ou la revendication 3, dans lequel l'anticorps de piégeage est immobilisé par fixation indirecte à la surface d'une plaque.

5. Procédé selon la revendication 1, dans lequel l'antigène est immobilisé par fixation indirecte à une surface.

6. Procédé selon l'une quelconque des revendications précédentes, dans lequel la nucléoprotéine est exprimée par une cellule transfectée par une séquence d'ADN recombiné comprenant un promoteur polyhédrine de baculovirus lié fonctionnellement à une molécule d'ADN codant la nucléoprotéine.

7. Procédé selon la revendication 6, dans lequel la cellule est Spodoptera frugiperda.

8. Procédé selon la revendication 6, dans lequel la cellule est transfectée par une séquence d'ADN recombiné comprenant le plasmide pACYM1S-MVN.

## Patentansprüche

1. Verfahren für einen Immunoassay zum Nachweis von masernspezifischem Immunglobulin in einer Humanserumprobe, das die Stufen umfaßt:
(i) Inkontaktbringen der Probe mit einem immobilisierten rekombinanten Masern-Nucleoproteinantigen unter Bedingun-gen, die eine Bindung zwischen dem Immunglobulin und dem immobilisierten Antigen zulassen; und
(ii) Nachweis der Anwesenheit von masernspezifischem Immunglobulin, das an das immobilisierte Antigen gebunden ist.

2. Verfahren für einen Capture-Immunoassay zum Nachweis von masernspezifischem IgM-Immunglobulin in einer Humanserumprobe, das die Stufen umfaßt:
(i) Inkontaktbringen der Probe mit immobilisiertem, gereinigtem Anti-Human-IgM-Captureantikörper unter Bedingungen, die eine Bindung zwischen dem IgM und dem immobilisierten Antikörper zulassen;
(ii) Inkontaktbringen des abgefangenen IgM mit einem rekombinanten Masern-Nucleoprotein unter Bedingungen, die für eine Bindung zwischen dem Nucleoprotein und dem abgefangenen IgM hinreichend sind; und
(iii) Nachweis der Anwesenheit von Nucleoprotein, das an das abgefangene IgM gebunden ist.

3. Verfahren nach Anspruch 2, worin Stufe (iii) umfaßt: Inkontaktbringen des IgM-gebundenen Nucleoproteins mit einem monoklonalen, an einen nachweisbaren Marker gebundenen Anti-Nucleoprotein-Antikörper, unter Bedingungen, die für eine gegenseitige Bindung hinreichend sind, und Nachweis der gebundenen Markierung.

4. Verfahren nach Anspruch 2 oder Anspruch 3, worin der Capture-antikörper durch indirekte Fixierung auf einer Plattenoberfläche immobilisiert wird.

5. Verfahren nach Anspruch 1, worin das Antigen durch indirekte Fixierung auf einer Oberfläche immobilisiert wird.

6. Verfahren nach irgendeinem der vorhergehenden Ansprüche, worin das Nukleoprotein durch eine Zelle exprimiert wird, die mit einer rekombinanten DNA-Sequenz transfiziert ist, die einen Baculovirus-Polyhedrinpromotor umfaßt, der operabel an ein für das Nukleoprotein codierendes DNA-Molekül gekoppelt ist.

7. Verfahren nach Anspruch 6, worin die Zelle Spodoptera frugiperda ist.

8. Verfahren nach Anspruch 6, worin die Zelle mit einer rekombinanten DNA-Sequenz transfiziert ist, die Plasmid pAcYM1S-MVN umfaßt.
